# EUROPEAN PATENT SPECIFICATION

(11) **EP 2 375 479 B1**
(45) Date of publication and mention of the grant of the patent: **27.03.2013**
(21) Application number: 10380053.8
(22) Date of filing: 08.04.2010
(51) Int. Cl.: C07C 51/41, H01M 4/58, C07C 69/00

(54) **Composite negative material comprising a transition metal malonate**
Negatives Verbundstoffmaterial mit einem Übergangsmetallmalonat
Matériau composite négatif comprenant un malonate de métal de transition

(43) Date of publication of application: 12.10.2011
(73) Proprietor: Universidad De Córdoba, 14071 Córdoba (ES)
(72) Inventor: Tirado, Coello, 14012 Cordoba (ES); Lavela, Cabello, 14014 Cordoba (ES); Pérez, Vicente, 14006 Cordoba (ES); Alcantara, Roman, 14350 Cordoba (ES); Leon, Mohedano, 14011 Cordoba (ES); Aragon, Algarra, 14001 Cordoba (ES)
(74) Representative: Goulard, Sophie

(56) References cited:
- W.B.SCHAAP: "Polarography of iron oxalates, malonates and succinates", JOURNAL OF THE AMERICAN CHEMICAL SOCIETY, vol. 76, 20 November 1954 (1954-11-20), pages 5868-5872, XP002598595,
- SUN X G ET AL: "Electrochemical and impedance investigation of the effect of lithium malonate on the performance of natural graphite electrodes in lithium-ion batteries", JOURNAL OF POWER SOURCES, ELSEVIER SA, CH, vol. 195, no. 13, 18 January 2010 (2010-01-18), pages 4266-4271, XP026977018, ISSN: 0378-7753 [retrieved on 2010-01-18]
- KENZO NAGASE ET AL.: "Thermal dehydration reactions of bivalent transition metal malonate dihydrates in solid state", BULLETTIN OF THE CHEMICAL SOCIETY OF JAPAN, vol. 48, no. 11, 1 November 1975 (1975-11-01), pages 3184-3187, XP002598614,
- M. J. ARAGON ET AL.: "Synthesis and electrochemical reaction with lithium of mesoporous iron oxalate nanoribbons", INORGANIC CHEMISTRY, vol. 47, no. 22, 10 November 2008 (2008-11-10), pages 10366-10371, XP002614193,
- M.J. ARAGON ET AL.: "Cobalt oxalate nanoribbons as negative-electrode material for lithium-ion batteries", CHEMISTRY OF MATERIALS, vol. 21, 4 June 2009 (2009-06-04), pages 1834-1840, XP002614194,
- R.C.LORD: "An investigation of the double cobalt malonates", JOURNAL OF PHYSICAL CHEMISTRY, vol. 11, no. 3, 1 January 1907 (1907-01-01), pages 173-200, XP002614200,

## Description

The invention relates to lithium rechargeable batteries. More particularly, the invention relates to a low voltage material for the negative electrode of secondary rechargeable batteries, wherein the active material is based on a transition metal malonate.

Negative electrode materials for rechargeable lithium batteries are generally selected from carbon materials, and tin-based composite materials. Recent studies on silicon-based electrodes and conversion oxide electrodes are found in the literature, whereas high capacities have been reported. Some of the major drawbacks of these potentially applicable electrode materials are high irreversible capacities and moderate rate performances.

As referred to supercapacitors, rate performance can be improved by using materials with mixed faradaic and pseudocapacitive materials. The synthesis and pseudocapacitive characteristics of titanium dioxide thin films with an anatase structure were recently described in J. Am. Chem. Soc., 2009, 131, 1802. In this study, it was suggested that both a mesoporous morphology and the use of nanocrystals as the basic building blocks contribute to the development of metal oxide pseudo/super-capacitive properties. However, energy densities were penalized by the use of thin films.

In the Nature 407 (2000) 476, electrodes made of nanoparticles of transition-metal oxides (MO, where M is Co, Ni, Cu or Fe) are reported to show electrochemical capacities of 700 mAh.g⁻¹ at C/5 by a displacive redox reaction. However, capacity retention is limited and the extra capacity resulting from the reversible formation of a polymer layer impedes acceptable high-rate performance.

Later, international application WO0171833A1 discloses that a low-voltage rechargeable lithium battery cell which comprises at least one electrode member comprising a fine-particle chalcogenide of a transition metal (typically an oxide of Co, Cu, Fe, Ni, Mn, or Zn) can provide a stable battery cell having capacities more than doubled compared to those of prior rechargeable battery cells. However, capacity retention is poor and the extra capacity resulting from the reversible formation of a polymer layer impedes acceptable high-rate performance.

US2009225498A1 patent application describes an asymmetric hybrid capacitor with electrodes based on metal oxides. The lithium oxide is used as a cathode-active material, and metal oxide capable of accepting the Li ions supplied through the electrolyte is used as anode-active material, such that the Li ions of the same participate in the electrochemical reactions at both electrodes. As a result, it is possible to minimize the decrease in ionic conductivity during charge/discharge. Since metal oxide having high specific capacitance is used to form both electrodes, it is possible to maximize energy density and power density.

Finally, US2002102205A1 patent application describes rechargeable electrochemical cells, such as lithium batteries and asymmetric hybrid battery/supercapacitor systems, exhibiting exceptional specific capacity levels and stability over extended high-rate recharge cycling comprise nanostructure zero strain Li₄ Ti₅O₁₂ intercalation electrode material synthesized in a short duration process of annealing mixed TiO₂ and Li-source precursor compounds at about 800°C for a time of about 15-30 min which is not substantially longer than that required to effect maximum available reaction between the precursors, thereby substantially eliminating the growth of synthesized L1₄T1₅O₁₂ particles beyond nanostructure size. The process reduces by order of magnitude the time and energy required for synthesis of the active electrode material and fabrication of utilizing cell devices, and provides such nanostructure material which enables repeated, high-rate recharge cycling without loss of cell capacity or efficiency.

The objective of this invention to provide a negative electrode active material for lithium battery that produces total reversible capacity > 372 mAh.g⁻¹ in the potential range of 0.0-3.0 V.

Another objective of the invention is to provide high capacity retention upon prolonged cycling and high rate performance by the combined use of Faradaic and capacitive storage properties. This has been achieved by the direct use of transition metal malonates as the active electrode material.

A first object of the present invention is therefore a composite negative electrode material comprising a transition metal malonate of general formula (I) MC₃H₂O₄, wherein M represents one or more transition metals, a polymer binder and an agent providing electronic conduction.

The presence of transition metal(s) is chosen to allow a reversible conversion reaction that forms reduced products to the metallic state in a lithium oxysalt matrix. The conductive properties of the resulting nanoparticles of the transition metals and the isolating properties of the lithium oxysalt matrix, provide double layer storage capacity to the corresponding electrodes.

According to the invention, M is preferably one or more transition metals selected from the group consisting of Ti, V, Cr, Mn, Fe, Co, Ni, Cu, and Zn.

Among these elements given for M, Co and Fe are particularly preferred.

Preferably, the amount of malonate of formula (I) ranges from 30 to 90 wt % relative to the total weight of the composite negative electrode material, and more preferably from 60 to 70 wt %.

The binder agent is preferably a polymer binder which can be selected from the group consisting of carboxymethylcellulose (CMC), polyvinylidene fluoride (PVDF), polytetrafluoroethylene (PTFE), ethylene propylene diene monomer (EPDM) and mixtures thereof.

Preferably, the amount of binder agent ranges from 1 to 30 wt % relative to the total weight of the composite negative electrode material, and more preferably from 5 to 15 wt %.

The agent providing electronic conduction can be selected from the group consisting of carbon black, natural or synthetic graphite, cokes, and mixtures thereof.

Preferably, the amount of agent providing electronic conduction ranges from 10 to 50 wt % relative to the total weight of the composite negative electrode material, and more preferably from 20 to 40 wt %.

Another object of the present invention is a negative electrode comprising the above-described composite negative electrode material, said material being supported on a current collector.

The current collector is generally in the form of a foil, such as for example in the form of a cupper, nickel or steel foil. The use of a cupper foil is particularly preferred.

An additional object of the invention is a lithium battery comprising a positive electrode, an electrolyte and a negative electrode, and wherein the anode is a negative electrode according to the invention and as defined above.

The lithium battery of the invention has a total capacity of at least 80 mAh.g⁻¹ in the first cycle in the range 2.5-5.2 V and a capacity of the negative electrode above 372 mAh.g⁻¹ in the low voltage region with a capacity retention of more than 85 % after 40 cycles.

The positive electrode is composed of a current collector supporting a positive active material, said material being able to reversibly insert lithium ions at a higher potential than that of the negative electrode material. The material of the positive electrode is generally in the form of a composite material comprising an active material for a positive electrode, a binder and an agent providing electronic conduction.

The binder and the agent providing electronic conduction can be chosen among those mentioned above for the negative electrode.

According to a preferred embodiment of the present invention, the positive active material is selected from the group consisting of:
- lithium-transition metal oxides, in particular lithium-transition metal layered oxides of the formula LiM¹O₂, wherein M¹ is at least one metal selected from the transition metals.
- lithium-transition metal phosphates;
- lithium-transition metal silicates.

Among lithium-transition metal layered oxides of formula LiM¹O₂, those in which M¹ is selected in the group consisting Mn, Fe, Co and Ni are particularly preferred.

The electrolyte is either a solid electrolyte (polymer or vitreous) or a separator impregnated with a liquid electrolyte.

The electrolyte is a lithium salt in solution in a solvent.

According to a preferred embodiment of the present invention, the lithium salt LiPF₆ is preferably chosen.

The solvent for the electrolyte can be a liquid solvent comprising one or several aprotic polar compounds selected in the group consisting of linear or cyclic carbonates, linear or cyclic ethers, linear or cyclic esters, sulfamides and nitriles.

According to a preferred embodiment of the present invention, the solvent is a mixture of two carbonates selected in the group consisting of ethylene carbonate, propylene carbonate, dimethyl carbonate, diethyl carbonate and methyl ethyl carbonate. A mixture of ethylene carbonate and diethyl carbonate is particularly preferred.

The solvent for the electrolyte can also be a solvating polymer. As examples of solvating polymers, it can be mentioned poly(ethyleneoxide)-based polyether polymers having a linear, block or comb-type branched structure, optionally cross-linked; copolymers containing an ethyleneoxide, a propylene oxide or an allylglycidylether moiety; polyphosphazenes, networks of polyethylene glycol cross-linked with isocyanates; and ethyleneoxide and epichlorhydrin copolymers such as those described in FR-9712952.

In addition to the solvating polymer, the solvent for the electrolyte may further comprise a polar non-solvating polymer comprising units containing at least one heteroatom selected from the group consisting of sulphur, oxygen, nitrogen and fluorine. The polar non-solvating polymer can be chosen among acrylonitrile homo- and copolymers, fluorovinylidene homo- and copolymers, and N-vinylpyrrolidone homo- and copolymers. The polar non-solvating polymer can also be chosen among polymers bearing ionic moieties, such as for example polyperfluoroether sulfonate (such as Nafion®) or a polystyrene sulfonate salt.

The transition metal malonate of general formula (I) can be prepared either by a simple precipitation method, or by a reverse micelle method.

According to the simple precipitation method, aqueous solutions containing inorganic or organic salts of M, and aqueous solutions of a compound of general formula (II) YₙC₃H₄O₄·yH₂O (Y being H, NH₄, or an alkali metal, 0 ≤ y) are prepared separately and then mixed.

The precipitate product has the MC₃H₂O₄·xH₂O stoichiometry, with x > 0 and has to be dehydrated, for example by thermal treatment, before being used in the manufacturing of the negative electrode.

Dehydration is important because the product obtained in hydrated form is not electro attractive towards lithium.

The reverse micelle method for the preparation of a transition metal malonate of general formula MC₃H₂O₄ (I), wherein M is as defined above, comprises the steps consisting of:
1a) preparing a first aqueous solution AS1 by totally dissolving in water at least one compound of general formula (II):

   YₙC₃H₂O₄·_{y}H₂O (II)

   wherein Y is H, NH₄, or an alkali metal, and 0 ≤ y;
1b) separately preparing a second aqueous solution AS2 by totally dissolving in water at least one organic or inorganic salt of M, M being a transition metal;
2a) adding to solution AS1 at least one surfactant, at least one co-surfactant and at least one hydrocarbon phase, to obtain a biphasic system BS1 comprising reverse micelles;
2b) adding to solution AS2 at least one surfactant, at least one co-surfactant and at least one hydrocarbon phase, to obtain a biphasic system BS2 comprising reverse micelles;
3) mixing the biphasic system BS1 with the biphasic system BS2 and heating the resulting mixture thereof to form a precipitate having the general formula (I'):

   MC₃H₂O₄·xH₂O (I')

   wherein M has the same meaning as in formula (I) above and x > 0;
4) recovering said precipitate;
5) drying said precipitate;
6) dehydrating said precipitate, by thermal treatment, to obtain the corresponding transition metal malonate of formula (I).

According to independently preferred embodiments:
- M is Fe or Co;
- the amount of compound of formula (II) in the first aqueous solution AS1 ranges from 1 to 30 wt % relative to the total weight of the solution AS1, and more preferably from 5 to 15 wt %;
- the amount of the said at least one organic or inorganic salt of M in the second aqueous solution AS2 ranges from 1 to 30 wt % relative to the total weight of the solution AS2, and more preferably from 5 to 15 wt %;
- the volume ratio AS1/AS2 ranges from 0.8 to 1.2;
- the surfactant is chosen among ionic surfactants with variable alkyl chain length, and more preferably among cationic surfactant in which the alkyl chain has from 4 to 24 carbon atoms. Examples of such surfactants are cetyltrimethylammonium bromide (CTAB), lauryldimethylamine-oxide (LDAO) and didodecyldimethyl-ammonium bromide (DDAB). Among those surfactants, CTAB is particularly preferred;
- the co surfactant is chosen among alcohols having at least 3 carbon atoms.
   Examples of co surfactants are butanol, pentanol, hexanol, heptanol, etc. Among those co surfactants, hexanol is particularly preferred;
- the hydrocarbon phase is selected from the group consisting of water insoluble cyclic or linear chain hydrocarbons or esters or ethers. As examples of hydrocarbon phase, it can be mentioned isooctane, cyclohexane, n-hexane, etc. Isooctane is particularly preferred.

As examples of compounds of formula (II), it can be mentioned sodium malonate Na₂C₃H₂O₄, malonic acid H₂C₃H₂O₄, ammonium malonate (NH₄)₂C₃H₂O₄, etc. Among these compounds, Na₂C₃H₂O₄, is particularly preferred.

The salts of M can for example be chosen among sulphates, nitrates, chlorides, etc. Among these salts, sulphates (MSO₄·xH₂O) and nitrates (M(NO₃)₂·xH₂O) are particularly preferred.

According to a preferred embodiment, steps 1a), 1b), 2a) and 2b), i.e. preparation of AS1 and AS2 and further of BS1 and BS2, are carried out under argon atmosphere.

The heating of the mixture of BS1 and BS2 in step 3) is preferably carried out at a temperature ranging from about 50 to 80°C, and more preferably from about 55 to 65°C.

According to a particular embodiment of the reverse micelle method, the precipitate recovered at the end of step 4), is preferably washed with an organic solvent, for example a lower alcohol such as methanol, chloroform, or a mixture thereof, before being dried in step 5).

The drying of step 5) is preferably performed under vacuum, and at a temperature ranging from about 0 to about 100°C, and more preferably from about 70 to about 80 °C.

The precipitate of formula (I') is obtained at the end of step 5) in hydrated form and may not be electroactive towards lithium. It is why it has to be dehydrated during step 6) to yield to an anhydrous compound of formula (I) as described above. The thermal treatment of step 6) is preferably performed at a temperature ranging from about 100 to 400°C. It is preferably carried out under vacuum or inert gas to avoid oxidation of M. The active compound of formula (I) is then cooled to room temperature.

The transition metal malonate of formula (I) obtained according to the above detailed method is is characterized in that it exhibits a low particle size ranging (micrometric and submicrometric particles) from about 10 to about 2,000 nm. Transition metal malonates of formula (I) in which M is selected from the group consisting of Ti, V, Cr, Mn, Fe, Co, Ni, Cu, Zn, are obtained in the form of nanoribbons with 10-1000 nm wide.

Additional features of the invention are disclosed in the following illustrating examples and accompanying figures:
- Figure 1 shows the X-ray diffraction patterns of MC₃H₂O₄·2H₂O (M = Fe and Co; intensity in arbitrary units as a function of scattering angle in degrees);
- Figure 2 shows transmission electron micrograph of FeC₃H₂O₄ obtained after dehydrating the reverse micelles product;
- Figure 3 shows the X-ray diffraction patterns of dehydrated products with FeC₃H₂O₄ and CoC₃H₂O₄ composition, obtained by the dehydration of CoC₃H₂O₄·2H₂O and FeC₃H₂O₄·2H₂O solids, respectively, previously prepared by the reverse micelle method (intensity in arbitrary units as a function of scattering angle in degrees);
- Figure 4 shows capacity (mAh.g⁻¹) versus cycle number of dehydrated cobalt malonate obtained by the reverse micelle method; the values were obtained in galvanostatic mode (C rate) (C = 1 Li/M/h; nC rate means an insertion of nLi per mole per hour) in a range 0.0-3.0 V;
- Figure 5 shows a comparison of capacities versus cycle number between (a) dehydrated iron malonate obtained by reverse micelles, (b) dehydrated cobalt malonate obtained by reverse micelles (c) dehydrated iron malonate obtained by simple precipitation, and (d) iron oxalate prepared by the reverse micelles method. The values were obtained in galvanostatic mode (C rate) (C = 1 Li/M/h; nC rate means an insertion of nLi per mole per hour) in a range 0.0-3.0 V.

### Example 1

### Preparation of iron malonate and cobalt malonate by the reverse micelle method

This example describes the preparation of iron malonate and of cobalt malonate by the reverse micelle method and emphasizes the effect of dehydration.

### 1) Preparation of iron malonate (FeC₃H₂O₄) by the reverse micelle method

FeC₃H₂O₄·2H₂O was first synthesised using the reverse micelle method.

Two biphasic systems (BS1 and BS2) were obtained under argon atmosphere using the same system for both BS1 and BS2: cetyl-trimethylammonium bromide (CTAB) (16.76 g), hexanol (16.93 mL), isooctane (85.9 mL) and aqueous phase (10 mL).

0.3 M solutions of iron (II) sulphate, and sodium malonate were used as the aqueous phases in BS1 and BS2, respectively. Both BS1 and BS2 were stirred and sonicated. Both BS1 and BS2 were then slowly mixed and stirred overnight at 80°C. The resulting precipitate was separated by centrifugation, washed with a 1:1 mixture of methanol and chloroform and heated to dryness at 80°C under vacuum.

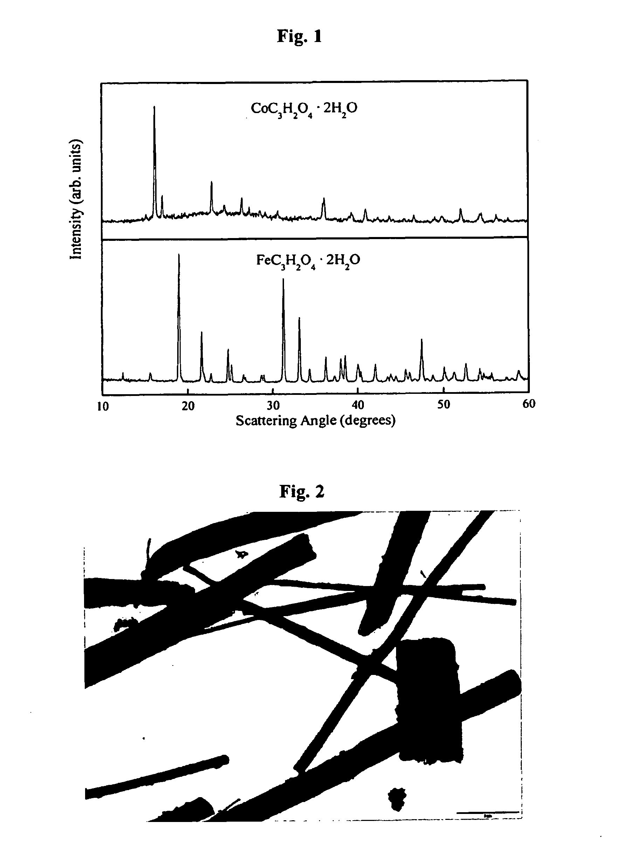

Figure 1 shows the X-ray diffraction patterns of MC₃H₂O₄·2H₂O (M = Fe and Co; intensity in arbitrary units as a function of scattering angle in degrees). For iron malonate, figure 1 shows an orthorhombic P2₁2₁2 structure with unit cell parameters a = 0.8229₂ nm, b = 0.9451 g nm, and c = 0.7285₇ nm.

### 2) Preparation of cobalt malonate (CoC₃H₂O₄) by the reverse micelle method

CoC₃H₂O₄·2H₂O was synthesis by the said method than FeC₃H₂O₄·2H₂O, but replacing iron (II) sulphate by cobalt (II) nitrate.

As can be seen on Figure 1, X-ray diffraction analysis of C_{O}C₃H₂0₄·2H₂O shows a monoclinic 12/m structure with unit cell parameters a = 1.100, nm, b = 0.7413₈ nm, c = 0.7317₆ nm, and β = 95.0₅°.

Thus, in the transition metal malonates of formula (I), the structure depends on the chemical composition of the solids.

CoC₃H₂O₄·2H₂O and FeC₃H₂O₄·2H₂O were then dehydrated at 200°C under vacuum for 5 h to lead to CoC₃H₂O₄ and FeC₃H₂O₄.

Figure 2 shows transmission electron micrograph of FeC₃H₂O₄ obtained after dehydrating the reverse micelles product FeC₃H₂O₄. FeC₃H₂O₄ showed micrometric and submicrometric particles with elongated shapes. Nanoribbons with 10-1000 nm wide are observed with the reverse micelle method.

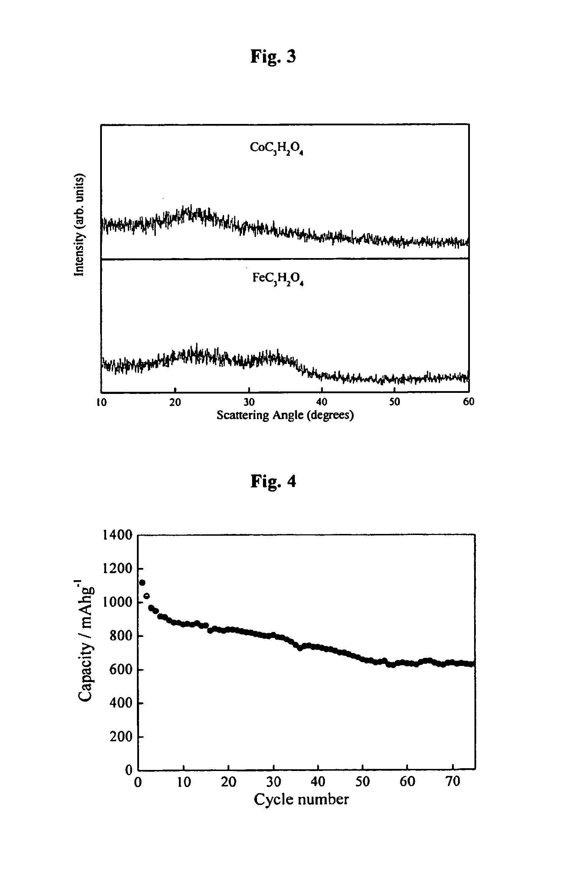

Figure 3 shows the X-ray diffraction pattern of dehydrated products with CoC₃H₂O₄ and FeC₃H₂O₄ composition, obtained by the dehydration of CoC₃H₂O₄-2H₂O and FeC₃H₂O₄·2H₂O solids, respectively, previously prepared by the reverse micelles method. On that figure, intensity in arbitrary units is a function of scattering angle in degrees.

FeC₃H₂O₄ was also prepared by a simple precipitation process, i.e. by directly mixing AS1 and AS2, followed by a similar stirring, centrifugation and drying process as in the synthesis by the reverse micelle method. Irregularly shaped particles of several micrometers (not shown) were visible for the FeC₃H₂O₄ obtained by the simple precipitation process. The average particle size was larger than in the micellar product.

### Example 2

### Preparation of cobalt malonate electrode and its cycling in a lithium test cell.

Cobalt malonate as prepared by the reverse micelle method in example 1, was used in this example to prepare electrode that were tested in lithium cells.

The electrochemical test was carried out in a two-electrode cell. The anodes were prepared by blending the powdered active material (60 %) with carbon black (30 %) and PVDF (10 %) dissolved in n-methyl-pyrrolidone as a binder. The slurry was then cast onto cupper foil and dried at 120 °C for 2 h. A lithium foil was used as counter electrode. The electrolyte was a solution of ethylene carbonate and diethyl carbonate (1:1) including 1 M of LiPF₆.

The corresponding results are shown on Figure 4 (capacity (mAh.g⁻¹) versus cycle number). The values were obtained in galvanostatic mode (C rate) (C = 1 Li/M/h; nC rate means an insertion of nLi per mole per hour) in a range 0.0-3.0 V.

These results show that the capacity of the first discharge was close to 1100 mAh.g⁻¹, which decreased during 10 cycles to 880 mAh.g⁻¹. After 75 cycles the value was 620 mAh.g⁻¹. Thus, the malonate in this example provides capacities significantly higher than graphite (372 mAh.g⁻¹) on prolonged cycling.

### Example 3

This example compares the cycling performance of iron malonate, cobalt malonate, iron oxalate and cobalt oxalate in lithium test cells.

Cobalt malonate and iron malonate were prepared by the reverse micelles method as in examples 1 and 2. Iron oxalate and cobalt oxalate were prepared as described in the literature (Inorganic Chemistry, 47, n° 22, 2008 "Synthesis and Electrochemical Reaction with Lithium of Mesoporous Iron Oxalate Nanoribbons" and Chem. Mater. 2009, 21, 1834-1840 "Cobalt Oxalate Nanoribbons as Negative-Electrode Material for Lithium-ion Batteries").

Electrodes containing the anhydrous malonates and oxalates prepared by the reverse micelle method were tested in lithium cells. The electrochemical test was carried out in a two-electrode cell. The cells were prepared as described in example 2.

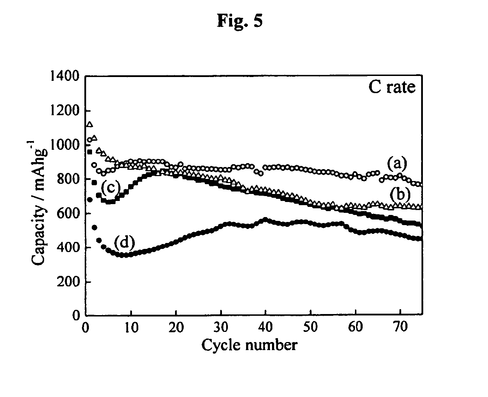

Figure 5 shows a comparison of capacities versus cycle number between (a) dehydrated iron malonate obtained by reverse micelles, (b) dehydrated cobalt malonate obtained by reverse micelles (c) dehydrated iron malonate obtained by simple precipitation, and (d) iron oxalate prepared by the reverse micelles method. The values were obtained in galvanostatic mode (C rate) (C = 1 Li/M/h; nC rate means an insertion of nLi per mole per hour) in a range 0.0-3.0 V.

The electrochemical results show that the capacities of the first discharge for iron malonate, cobalt malonate, iron oxalate and cobalt oxalate were close to 1020, 1100, 700, and 850 mAh.g⁻¹, respectively. After 75 cycles the values were close to 760, 620, 500, and 580 mAh.g⁻¹, for iron malonate, cobalt malonate, iron oxalate and cobalt oxalate, respectively. Thus, the malonates in this example provides capacities significantly higher than graphite (372 mAh.g⁻¹) and oxalates on prolonged cycling.

In conclusion, iron malonate behaves better than graphite, iron oxalate and cobalt malonate. Iron malonate prepared by the reverse micelles method behaves better than iron malonate prepared by a simple precipitation process.

## Claims

1. A composite negative electrode material comprising a transition metal malonate of general formula (I) MC₃H₂O₄, wherein M represents one or more transition metals, a polymer binder and an agent providing electronic conduction.

2. The composite negative electrode material according to claim 1, wherein M is one or more transition metals selected from the group consisting of Ti, V, Cr, Mn, Fe, Co, Ni, Cu, and Zn.

3. The composite negative electrode material according to claim 2, wherein M is Co or Fe.

4. The composite negative electrode material according to anyone of claims 1 to 3, wherein the amount of malonate of formula (I) ranges from 30 to 90 wt % relative to the total weight of the composite negative electrode material.

5. The composite negative electrode material according to anyone of claims 1 to 4, wherein the binder agent is selected from the group consisting of carboxymethylcellulose, polyvinylidene fluoride, polytetrafluoroethylene, ethylene propylene diene monomer and mixtures thereof.

6. The composite negative electrode material according to anyone of claims 1 to 5, wherein the amount of binder agent ranges from 1 to 30 wt % relative to the total weight of the composite negative electrode material.

7. The composite negative electrode material according to anyone of claims 1 to 6, wherein the agent providing electronic conduction is selected from the group consisting of carbon black, natural or synthetic graphite, cokes, and mixtures thereof.

8. The composite negative electrode material according to anyone of claims 1 to 7, wherein the amount of agent providing electronic conduction ranges from 10 to 50 wt % relative to the total weight of the composite negative electrode material.

9. A negative electrode comprising a composite negative electrode material as defined in anyone of claims 1 to 8, said material being supported on a current collector.

10. The negative electrode according to claim 9, wherein the current collector is in the form of a copper, nickel or steel foil.

11. A lithium battery comprising a positive electrode, an electrolyte and a negative electrode, wherein the anode is a negative electrode as defmed in any of claims 9 and 10.

12. The lithium battery according to claim 11, wherein the positive electrode is composed of a current collector supporting a positive active material, said positive active material being selected from the group consisting of lithium-transition metal oxides, lithium-transition metal phosphates, and lithium-transition metal silicates.

13. The lithium battery according to claim 11 or 12, wherein the electrolyte is a lithium salt in solution in a solvent.

## Patentansprüche

1. Negatives Verbundstoffelektrodenmaterial, das ein Übergangsmetallmalonat der allgemeinen Formel (I) MC₃H₂O₄, wobei M ein oder mehrere Übergangsmetalle darstellt, ein Polymerbindemittel und ein Mittel umfasst, das elektronische Leitung bereitstellt.

2. Negatives Verbundstoffelektrodenmaterial nach Anspruch 1, wobei M ein oder mehrere Übergangsmetalle ist, ausgewählt aus der Gruppe, bestehend aus Ti, V, Cr, Mn, Fe, Co, Ni, Cu und Zn.

3. Negatives Verbundstoffelektrodenmaterial nach Anspruch 2, wobei M Co oder Fe ist.

4. Negatives Verbundstoffelektrodenmaterial nach einem der Ansprüche 1 bis 3, wobei die Menge an Malonat der Formel (I) im Bereich von 30 bis 90 Gew.-% in Bezug auf das Gesamtgewicht des negativen Verbundstoffelektrodenmaterials liegt.

5. Negatives Verbundstoffelektrodenmaterial nach einem der Ansprüche 1 bis 4, wobei das Bindemittel aus der Gruppe, bestehend aus Carboxymethylcellulose, Polyvinylidenfluorid, Polytetrafluorethylen, Ethylenpropylendienmonomer und Mischungen davon, ausgewählt ist.

6. Negatives Verbundstoffelektrodenmaterial nach einem der Ansprüche 1 bis 5, wobei die Menge an Bindemittel im Bereich von 1 bis 30 Gew.-% in Bezug auf das Gesamtgewicht des negativen Verbundstoffelektrodenmaterials liegt.

7. Negatives Verbundstoffelektrodenmaterial nach einem der Ansprüche 1 bis 6, wobei das Mittel, das elektronische Leitung bereitstellt, aus der Gruppe, bestehend aus Ruß, natürlichem oder synthetischen Graphit, Koks und Mischungen davon, ausgewählt ist.

8. Negatives Verbundstoffelektrodenmaterial nach einem der Ansprüche 1 bis 7, wobei die Menge an dem Mittel, das elektronische Leitung bereitstellt, im Bereich von 10 bis 50 Gew.-% in Bezug auf das Gesamtgewicht des negativen Verbundstoffelektrodenmaterials liegt.

9. Negative Elektrode, umfassend ein negatives Verbundstoffelektrodenmaterial nach einem der Ansprüche 1 bis 8, wobei das Material auf einem Stromabnehmer getragen wird.

10. Negative Elektrode nach Anspruch 9, wobei der Stromabnehmer in Form von Kupfer-, Nickel- oder Stahlfolie vorliegt.

11. Lithiumbatterie, umfassend eine positive Elektrode, einen Elektrolyten und eine negative Elektrode, wobei die Anode eine negative Elektrode nach einem der Ansprüche 9 und 10 ist.

12. Lithiumbatterie nach Anspruch 11, wobei die positive Elektrode aus einem Stromabnehmer zusammengesetzt ist, der ein positives aktives Material trägt, wobei das positive aktive Material aus der Gruppe, bestehend aus Lithiumübergangsmetalloxiden, Lithiumübergangsmetallphosphaten und Lithiumübergansmetallsilicaten, ausgewählt ist.

13. Lithiumbatterie nach Anspruch 11 oder 12, wobei der Elektrolyt ein Lithiumsalz in Lösung in einem Lösungsmittel ist.

## Revendications

1. Matériau composite d'électrode négative comprenant un malonate d'un métal de transition de formule générale (I) MC₃H₂O₄ dans laquelle M représente un ou plusieurs métaux de transition, un liant polymère et un agent conférant une conductivité électronique.

2. Matériau composite d'électrode négative selon la revendication 1, **caractérisé en ce que** M représente un ou plusieurs métaux de transition choisis dans le groupe constitué de Ti, V, Cr, Mn, Fe, Co, Ni, Cu, et Zn.

3. Matériau composite d'électrode négative selon la revendication 2, **caractérisé en ce que** M est Co ou Fe.

4. Matériau composite d'électrode négative selon l'une quelconque des revendications 1 à 3, **caractérisé en ce que** la quantité de malonate de formule (I) varie de 30 à 90% en masse par rapport à la masse totale du matériau composite d'électrode négative.

5. Matériau composite d'électrode négative selon l'une quelconque des revendications 1 à 4, **caractérisé en ce que** l'agent est sélectionné dans le groupe constitué de la carboxyméthylcellulose, du fluorure de polyvinylidène, du polytétrafluoroéthylène, du monomère éthylène propylène diène et de leurs mélanges.

6. Matériau composite d'électrode négative selon l'une quelconque des revendications 1 à 5, **caractérisé en ce que** la quantité d'agent liant varie de 1 à 30% en masse par rapport à la masse totale du matériau composite d'électrode négative.

7. Matériau composite d'électrode négative selon l'une quelconque des revendications 1 à 6, **caractérisé en ce que** l'agent conférant une conductivité électronique est sélectionné dans le groupe constitué du noir de carbone, du graphite naturel ou synthétique, de cokes, et de leurs mélanges.

8. Matériau composite d'électrode négative selon l'une quelconque des revendications 1 à 7, **caractérisé en ce que** la quantité d'agent conférant une conductivité électronique varie 10 à 50 % en masse par rapport à la masse totale du matériau composite d'électrode négative.

9. Electrode négative comprenant un matériau composite d'électrode négative tel que défini dans l'une quelconque des revendications 1 à 8, ledit matériau étant supporté sur un collecteur de courant.

10. Electrode négative selon la revendication 9, **caractérisée en ce que** le collecteur de courant est sous la forme d'une feuille de cuivre, de nickel ou d'acier.

11. Batterie au lithium comprenant une électrode positive, un électrolyte et une électrode négative, dans laquelle l'anode est une électrode négative telle que définie dans l'une quelconque des revendications 9 et 10.

12. Batterie au lithium selon la revendication 11, dans laquelle l'électrode positive est composée d'un collecteur de courant supportant un matériau d'électrode positive, ledit matériau d'électrode positive étant sélectionné dans le groupe constitué d'oxydes de lithium et d'un métal de transition, de phosphate de lithium et d'un métal de transition, et de silicates de lithium et d'un métal de transition.

13. Batterie au lithium selon la revendication 11 ou 12, dans laquelle l'électrolyte est un sel de lithium en solution dans un solvant.
